Europäisches Patentamt

European Patent Office

Office européen des brevets

(11) Veröffentlichungsnummer: **0 351 717**

**A2**

(12) **EUROPÄISCHE PATENTANMELDUNG**

(21) Anmeldenummer: **89112826.6**

(22) Anmeldetag: **13.07.89**

(51) Int. Cl.4: **C12N 15/56 , C12N 15/75**

Der (Die) Mikroorganismus (Mikroorganismen) ist (sind) bei DSM unter der (den) Nummer(n) 3406, 3407, 641, 5213 und 5214 hinterlegt worden.

(30) Priorität: **21.07.88 DE 3824827**

(43) Veröffentlichungstag der Anmeldung: **24.01.90 Patentblatt 90/04**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Okada, Jungo**
**299-7, Kouzu**
**Yachiyo-shi(Takatsu) Chiba 276(JP)**
Erfinder: **Hänggi, Urs**
**Karl-Kautsky-Weg 42**
**A-4040 Linz(AT)**
Erfinder: **Berger, Harald, Dr.**
**Alt Pempelfort 11 a**
**D-4000 Düsseldorf(DE)**
Erfinder: **Gamon, Konrad, Dr.**
**Herderstrasse 65**
**D-4000 Düsseldorf 1(DE)**
Erfinder: **Markgraf, Martina**
**Peterstrasse 11**
**D-5024 Pulheim 4(DE)**

(54) **Plasmide zur Erzeugung von alpha-Amylase in Bacillus.**

(57) In Bacillus exprimierbares Bacillus Plasmid, enthaltend einen Promotor für die Expression von Proteinen und in funktionsfähiger Weise damit verknüpft das Strukturgen eines Enzyms, dadurch gekennzeichnet, daß es einen Protease-Promotor verknüpft mit einem Amylase-Strukturgen enthält.

EP 0 351 717 A2

Fig 3

## Plasmide zur Erzeugung von alpha-Amylase in Bacillus

Die Erfindung betrifft Plasmide, mit deren Hilfe Amylasen in Bacillus unter den gleichen Bedingungen wie Subtilisin Carlsberg erzeugt werden können.

Unter Amylasen versteht man Enzyme, die in der Lage sind, die alpha-1 -4- und/oder die alpha-1-6-Bindung der Stärke zu spalten.

Amylasen werden großtechnisch zur Modifizierung von Stärke eingesetzt, können jedoch auch in Wasch- und Reinigungsmitteln zur Beseitigung stärkehaltiger Verschmutzungen verwendet werden. In technischen Mengen ist beispielsweise eine thermostabile alpha-Amylase aus Bacillus licheniformis erhältlich. Diese alpha-Amylase wird üblicherweise durch Fermentation gewonnen. Sie wird hauptsächlich in der späten exponentiellen Wachstumsphase von Bacillus licheniformis secerniert. Zur verbesserten Herstellung dieses Enzyms hat man bereits versucht, durch Zusätze zum Medium die Ausbeute zu erhöhen oder durch Selektion überproduzierende Mutanten zu gewinnen. Auch wurde versucht, durch Amplifikation des klonierten Gens in Bacillus subtilis Stämme mit erhöhter Produktionsfähigkeit zu gewinnen (Piggott et al, Biochem., Biophys., Res., Comm. 122, 175 (1984); G. Hepp et al, Gene 23, 267, (1983)).

Ein wesentlicher Nachteil aller genannten Verfahren besteht darin, daß sie der sogenannten Katabolitrepression durch Glucose unterliegen. Das heißt, daß Glucose, die durch die Einwirkung der alpha-Amylase auf die Stärke im Fermentationsmedium entsteht, die Synthese weiterer alpha-Amylase unterdrückt.

Diesem Umstand trägt ein Verfahren Rechnung, das in der europäischen Patentanmeldung 0 205 371 beschrieben wird. Dort wird vorgeschlagen, das Gen für die thermostabile alpha-Amylase aus B. licheniformis mit Teilen des Levan-Saccharase-Gens von Bacillus subtilis zu verknüpfen (sacR-sacB'-SC-SDamy). In diesem Konstrukt wird die alpha-Amylase in Bacillus subtilis in der exponentiellen Wachstumsphase in Gegenwart von Zucker ohne Katabolitrepression produziert.

Die Bedingungen, unter denen der Levan-Saccharase-Promotor aktivierbar ist, sind jedoch in der großtechnischen Fermentation nicht untersucht und wahrscheinlich schwer zu beherrschen. Darüber hinaus eröffnet das Verfahren der europäischen Patentanmeldung 0 205 371 nicht die Möglichkeit, gleichzeitig die für technische Zwecke besonders gewünschten Mischungen aus Protease und Amylase unter den für beide Systeme optimalen Bedingungen zu produzieren.

Aufgabe der Erfindung ist es somit, die Expression eines Amylase-Gens, beispielsweise des Gens für die thermostabile alpha-Amylase aus B. licheniformis so zu verändern, daß große Mengen Enzyme mit Hilfe der etablierten, bei der Proteaseherstellung angewendeten Verfahren, überproduziert werden können.

Gegenstand der Erfindung ist daher ein in Bacillus licheniformis exprimierbares Bacillus Plasmid, enthaltend einen Promotor für die Expression von Proteinen und - in funktionsfähiger Weise damit verknüpft - das Strukturgen eines Enzyms, dadurch gekennzeichnet, daß es einen Protease-Promotor verknüpft mit einem Amylase-Strukturgen enthält.

Weitere Gegenstände der Erfindung sind Mikroorganismen, die mit derartigen Plasmiden transformiert sind, sowie ein Verfahren zur Herstellung von Amylase, gewünschtenfalls in Abmischung mit Protease, unter den bei alkalischen Proteasen üblichen Bedingungen.

Ein weiterer Gegenstand der Erfindung ist ein Verfahren zur Erzeugung einer alpha-Amylase, gewünschtenfalls in Abmischung mit alkalischer Protease, bei dem ein Mikroorganismus der Gattung Bacillus licheniformis oder Bacillis subtilis, welcher Plasmide mit der Fähigkeit, alpha-Amylase in der stationären Wachstumsphase zu erzeugen enthält, unter den für die Erzeugung von Subtilisin Carlsberg üblichen Bedingungen kultiviert und das exocellulär erzeugte Enzym abgetrennt und aufgearbeitet wird, wobei das in Bacillus exprimierbare Plasmid einen Promotor für die Expression von Proteinen und in funktionsfähiger Weise damit verknüpft das Strukturgen eines Enzyms enthält, dadurch gekennzeichnet, daß in dem im Mikroorganismus vorhandenen Plasmid ein Protease-Promotor verknüpft mit einem Amylase-Strukturgen vorhanden ist.

In ihrer allgemeinsten Ausführungsform betrifft die Erfindung ein Plasmid, das aus einem üblichen, bacillusgängigem Plasmidteil besteht, der in eine Schnittstelle eingefügt einen Protease-Promotor, insbesondere den Promotor der Protease Subtilisin-Carlsberg aus Bacillus licheniformis - und damit in funktionsfähiger Weise verknüpft ein Amylase-Gen, insbesondere das alpha-Amylase-Gen von Bacillus licheniformis, enthält. Das eingefügte Stück kann weiterhin Extrasequenzen ohne spezifische Expressionsfähigkeit enthalten; es muß die zur Funktionsfähigkeit nötigen weiteren DNA-Stücke wie beispielsweise Pro/Prä-Sequenzen, Start- und Stop-Codons und dergleichen mit beinhalten.

Das Ausgangsplasmid sollte weiterhin ein oder mehrere Resistenzmarker und ein oder mehrere Schnittstellen für übliche Restriktionsenzyme enthalten. Dabei kann das Konstrukt aus Protease-Promotor und Amylase-Gen in eine Schnittstelle eines Enzyms oder in eine durch Schneiden mit zwei Enzymen erzeugte Schnittstelle eingepaßt werden.

Zur Erzeugung der erfindungsgemäßen Plasmide kann der Fachmann beispielsweise von an sich bekannten Plasmiden ausgehen, die Promotor und Strukturgen der Protease, beispielsweise der alkalischen Protease Subtilisin Carlsberg, enthalten. Als weitere Komponente kann von Plasmiden ausgegangen werden, die das Strukturgen der gewünschten Amylase enthalten. Im Falle des Protease-Plasmids wird vor dem Start-Codon des Strukturgens der Protease durch gerichtete Mutagenese eine Enzymschnittstelle eingefügt. Diese Operation wird vorzugsweise in bekannter Weise in E. coli ausgeführt. Auch im Falle der Amylase wird vor dem Start-Codon eine Enzymschnittstelle erzeugt, so daß das gesamte Amylase-Strukturgen, das gewünschtenfalls hinter dem Stop-Codon noch Extrasequenzen ohne spezifische Expressionsfähigkeit enthält, entnommen werden kann. Der Protease-Promotor kann dann in ein Plasmid eingefügt werden, in das dann durch Linearisierung und erneuter Ligierung das Amylase-Gen eingebracht werden kann.

Geeignete Ausgangsplasmide, die Promotor- und Strukturgen der Protease enthalten, sind bekannt und werden beispielsweise in der deutschen Offenlegungsschrift DE 35 27 913 beschrieben. Die dort genannten Plasmide leiten sich von allgemein zugänglichen Ausgangsplasmiden ab, welche über ein oder mehrere Resistenzmarker und Schnittstellen für Restriktionsenzyme verfügen. Die Ausgangsplasmide müssen für Bacillus geeignet sein. Derartige Ausgangsplasmide sind beispielsweise die folgenden: pBCE16, pC194, pUB110, pE194, pSA2100, pPL608, pBD64. Hinweise auf diese Plasmide finden sich in der Fachliteratur an den folgenden Stellen:

Bernhard K., Schrempf H., Goebel W., 1978, J. Bacteriol. 133:897-903

Gryczan, T.J., Contente, S., and Dubnau, D. 1978, J. Bacteriol. 134:318-323

Williams, D.M., Duvall, E.J. and Lovett, P.S. 1981, J. Bacteriol. 146:1162-1165

Gryczan, T., Shivakumar, A.G., and Dubnau, D., 1980, J. Bacteriol. 141:246-253

Ehrlich, S.D., Bursztyn-Pettegrew. H., Stroynowski, J., and Leaderberg. J. 1977 In "Recombinat Molecules: Impact on Science and Society, S. 69-80, Raven Press, New York

Weisblum, B. Graham, M.Y., Gryczan, T., and Dubnau, D. 1979, J. Bacteriol. 137:635-643

Bei der Auswahl geeigneter Ausgangsplasmide ist darauf zu achten, daß sie durch spezifische Spaltenzyme linearisierbar sind, ohne dabei ihre charakteristischen Eigenschaften zu verlieren. Unter charakteristischen Eigenschaften sind hier die Replikationsfähigkeit, die Resistenz und die Möglichkeit zur Expression in Bacillus zu verstehen. Gängige spezifische Spaltenzyme, mit denen geeignete Ausgangsplasmide linearisiert werden können, sind beispielsweise BamHI, EcoRI, HindIII und PstI.

Nach der Lehre der DE 35 27 913 ist es möglich, diese Ausgangsplasmide mit Restriktionsenzymen zu spalten, ein isoliertes doppelsträngiges DNA-Stück einzuführen, das Promotor- und Strukturgen einer Protease, insbesondere von Subtilisin Carlsberg enthält und wieder zum Ring zu schließen.

Ein besonders geeignetes Ausgangsplasmid ist das Bacillus Plasmid pBCE16, das sich mit BamHI linearisieren läßt und in das dann gemäß DE 35 27 913 das Subtilisin Carlsberg Gen eingefügt werden kann. Das so erhaltene Plasmid trägt die interne Bezeichnung pC50. Ein weiteres, sehr geeignetes Plasmid leitet sich in analoger Weise von dem Bacillus Ausgangsplasmid pUB110 ab.

Aus dem Protease-Plasmid pC50 können mit den Restriktionsenzymen AvaI und SstI Strukturgen inklusive Pre- und Prosequenzen, Start-und Stopcodons und Promotorregion der Protease und ein beiderseits vorhandenes, nicht näher definiertes Stück ohne spezifische Expressionsfähigkeit isoliert und in das E. coli-Plasmid pUC19, das mit XmaI und Sst geschnitten worden war, eingefügt werden. Das genannte E. coli-Plasmid ist erhältlich u.a. bei New England Biolabs, Boehringer Mannheim u.a. und beschrieben bei Yanisch-Perron, C. et al, Gene (1985) 33, 1, 103-119.

Das so erhaltene E. Coli-Plasmid (interne Bezeichnung pH9) kann mit den Restriktionsenzymen EcoRI und AvaI geschnitten werden. Die Schnittstelle findet sich im Strukturgen der Protease zum einen und oberhalb des Promotors zum anderen. Das isolierte doppelsträngige DNA-Stück kann in die EcoRI und AvaI bzw. XmaI Schnittstellen des E. Coli Plasmids pMa254 eingesetzt werden zu einem Plasmid mit der internen Bezeichnung pMG55.

Das Plasmid pMa254 ist ein M 13 Derivat von H.-J. Fritz, Max-Planck-Institut für Biochemie, Martinsried. Es ist besonders geeignet für die gerichtete Mutagenese. Andere geeignete Plasmide sind M 13 mp9 (Kramer, W. und Fritz, H.-J., München, 1987, Meth. Enzymol. 154, 350 - 367). Andere geeignete System für die gerichtete Mutagenese können z.B. von Boehringer, Mannheim, BioRad oder Amersham Buchler bezogen werden.

Unter gerichteter Mutagenese soll hier der gezielte Austausch von zwei oder mehr Basen gegen andere Basen verstanden sein, um dadurch eine Schnittstelle zu schaffen.

Im Rahmen der Erfindung gelang es durch die im Folgenden beschriebene Vorgehensweise in Position -3 und -2 vor dem Start-Codon ATG des Proteasegens GT in TC umzuwandeln:

AGT GAG TAA TGA

zu

AGT GAT CAA TGA

Es wurde so möglich, eine Schnittstelle für das Enzym BclI zu schaffen.

Die gerichtete Mutagenese mit dem Plasmid pMa254 kann analog W. Kramer et al., Nucleic Acids Research 12, 9441-56 durchgeführt werden. Benutzt wurde ein für die Änderung in dieser Position geeigneter Primer. Es entsteht ein Konstrukt mit der internen Bezeichnung pMG56.

Zur Isolierung des Strukturgens der Amylase geht der Fachmann zweckmäßigerweise von einem Plasmid aus, das dieses Amylasegen enthält. Ein derartiges Plasmid ist beispielsweise beschrieben in FR 2 537 602, FR 2 533 583 sowie bei Joyet et al 1984, FEMS Mikrobiol. Let. 21, 353-358.

Die Klonierung des Amylasegens wurde bis auf die unterschiedliche Selektionierung der Amylaseklone nach der Transformation in Bacillus subtilis BD393 (im Unterschied zu BR151 für die Proteaseklonierung) entsprechend der in der deutschen Offenlegungsschrift DE 35 27 913 beschriebenen Vorgehensweise für die Klonierung der ‚Protease Subtilisin Carlsberg vorgenommen. Die Selektionierung wurde auf HGP-Agarplatten (HGP-Agar: 1 % Pepton, 0,5 % NaCl, 0,5 % Hefeextrakt, 0,5 % Glucose, 1 % Agarose) mit einem 1 % Maisstärke enthaltendem HGP-Agaroverlay und 15 mg/l Tetracylin durchgeführt.

2 Klone, die das Amylasegen erhalten haben, konnten nach ca. 40 Stunden unter ca. 12600 Transformanten unmittelbar auf der Agarplatte identifiziert werden, da diese Kolonien im Gegensatz zum Amylase-negativen Wirtsstamm BD393 klare Höfe im ansonsten durch Stärke getrübten Agar hervorrufen.
Die Plasmid-DNA des stabilen Amylaseklons wurde, wie in obiger Offenlegungsschrift beschrieben, isoliert (pA1).

Zur Charakterisierung der pA1-DNA mittels verschiedener Restriktionsenzyme wurde einzeln und in Kombination mit BclI, EcoRI, PstI und SalI gespalten.

Zur Erzeugung der erfindungsgemäßen Plasmide wird das als Ergebnis der gerichteten Mutagenese erhaltene, aus dem Protease-Plasmid pC50 entwickelte Plasmid mit der Bezeichnung pMG56 mit den Enzymen EcoRI und BclI (für die neu entstandene Schnittstelle) geöffnet. Auf diese Weise wird das Reststrukturgen der Protease entfernt.

In den so entstandenen Expressionsvektor kann erfindungsgemäß das Strukturgen der Amylase eingesetzt werden.

Mit Hilfe der gerichteten Mutagenese wird eine Schnittstelle zwischen Amylasepromotor und Amylasegen erzeugt. So kann insbesondere eine Schnittstelle für BclI erzeugt werden, indem die Nucleotide in den Positionen -5 und -4 vor dem Startcodon der Amylase von GA zu TG geändert werden. Der Konstrukt trägt die interne Bezeichnung pJ02.

Zum Erhalt des Amylase-Strukturgens mit Hilfe des Restriktionsenzyms BclI kann jetzt das Amylase-Strukturgen aus z.B. Plasmid pJ02 entnommen und in das Plasmid pMG56, das vorher mit BclI gespalten wurde, eingesetzt werden. Es entstehen Plasmide, die das Amylase-Strukturgen im statistischen Mittel in richtiger bzw. in gegenläufiger Richtung zum Promotor enthalten (interne Bezeichnungen pJ03 und pJ04). Dabei wurde beobachtet, daß die Plasmide mit dem AmylaseStrukturgen in Leserichtung zum Promotor bereits in E. coli die Amylase in kleinen Mengen exprimieren.

Zur Transferierung des Konstrukts, bestehend aus Protease-Promotor und Amylase-Strukturgen in ein Bacillusplasmid kann zweckmäßigerweise mit EcoRI und BamHI gespalten werden. Dadurch entsteht eine isolierte DNA-Sequenz, enthaltend Promotor, Strukturgen, die weiteren für die Funktionsfähigkeit wichtigen Sequenzen (Pro-/ Präsequenzen, Start- und Stop-Codons) und auf beiden Seiten Extrasequenzen bis zu den Schnittstellen der beiden genannten Restriktionsenzyme. Die so erhaltene isolierte doppelsträngige DNA-Sequenz kann zwischen die EcoRI und die BamHI Schnittstelle des Bacillus Plasmids pUB110 ligiert werden. In alternativer Weise kann dasselbe Stück aber auch in pBCE16 eingebaut werden.

So erhaltene amylaseproduzierende Stämme der Gattung Bacillus, insbesondere Bacillus licheniformis, DSM 641, DSM 3406 oder DSM 3407 sind in der Lage, Amylase in großen Mengen und gewünschtenfalls im Gemisch mit Proteasen herzustellen. Zum Test auf Amylaseproduktion kann man die Stämme auf Screening-Platten wachsen lassen, die 1 % Stärke enthalten. Durch den Stärkeabbau läßt sich visuell die Aktivität der Stämme am Verschwinden der Trübung erkennen.

Mikroorganismen die die erfindungsgemäßen Plasmide enthalten, zeigen im Vergleich zu nicht transformierten oder mit pAI transformierten Organismen eine vielfach höhere Amylase-Produktion. Dabei wird die Amylase in der Stationären Wachstumsphase ausgeschieden. So erhält man im Falle des Plasmids pAI eine 4- bis 5-fache, im Falle von pJ05 und pJ06 eine 40-fach höhere Amylaseproduktion als in nicht transformierten Organismen. Transferiert man das Amylase-Plasmid in Stämme, die bereits Protease produzieren, so lassen sich Stämme erzeugen, die in ihrer exponentiellen Wachstumsphase unter Protease-Produktionsbedingungen sowohl das eine als auch das andere Enzym erzeugen. In diesem Fall wird durch Plasmide mit Amylasepromotor (z.B. pA1) eine 15-fach erhöhte Amylaseproduktion, durch Plasmide mit Proteasepromotor eine 50-fach höhere Amylaseproduktion erreicht.

## Beispiele

Zur Durchführung der Experimente können die verwendeten Plasmid DNA's durch eine Reihe verschiedener, dem Fachmann bekannten Methoden isoliert werden, die einzeln oder kombiniert durchgeführt wurden (z.B: Clewell, D. B.; Helinsky, D.R.; PNAS 62, 1159 (1969) oder Holmes, D. S.; Quigley, M.; Annual. Biochem.141 193 (1981)). Die gereinigten DNA's können sodann in geeignetem Puffer mittels Restriktionsenzymen gepalten werden.

Zur Einführung der gerichteten Mutagenesen mußte zunächst einzelsträngige DNA des zu mutagenisierenden Plasmids, z. B.: pMG55 isoliert werden. 7,5ml LB-Medium wurden mit 2,5 ml Übernachtkultur eines entsprechenden plasmidhaltigen Stammes, in diesem Fall E. coli BMH71-18/pMG55 und 100ul F1 Helferphagenlysat M13K07 (Titer 1x10 12) angeimpft und für 5h bei 150 upm und 37 °C inkubiert. 1.5ml dieser Kultur wurden in ein Eppendorf Gefäß überführt und 5 min bei maximaler Drehzahl in einer Eppendorf Zentrifuge zentrifugiert. 1ml des phagenhaltigen Überstands wurden vorsichtig abgenommen und erneut zentrifugiert. Dannach wurden 800μl Überstand mit 200μl 2.5m NaCl/20% PEG 6000 gemischt und für 15 min bei Raumtemperatur belassen. Nach Zentrifugation für 5 min wurde der Überstand möglichst vollständig abgenommen, das Phagenpellet in 110μl TE- Puffer (10mM Tris(r) HCl pH 8/1mM EDTA) aufgenommen und mit 50μl Tris(r)-gesättigtem Phenol ausgeschüttelt. 100μl der wässrigen Phase wurden nochmals mit 100μl Chloroform ausgeschüttelt und dannach aus 90μl wässriger Phase die DNA nach Zugabe von 10μl 3M Na-acetat pH 6, 300μl Ethanol und Inkubation für 1h bei -20 °C ausgefällt. Das nach Zentrifugation erhaltene DNA-haltige Pellet wurde mit 1ml 70% Ethanol gewaschen, unter Vakuum getrocknet und in 25μl TE Puffer resuspendiert.

Zur Herstellung der zur Mutagenese benötigten partiell einzelsträngigen DNA werden 0,1pmol lineare Vektor DNA mit 0,5pmol einzelsträngiger Plasmid DNA, in diesem Fall Eco R1 und BamH1 linearisierte pMC254 DNA und einzelsträngige pMG55 DNA in 35μl Volumen gemischt. Nach Zugabe von 5μl 1.5MKCl/100mM Tris(r)-HCl pH7.5 wurde für 4 min auf 100 °C erhitzt und anschließend 10min bei 65 °C inkubiert. Die Bildung von partiell doppelsträngiger DNA wurde auf einem Agarosegel überprüft.

Die zur Einführung der gewüschten Mutationen benötigten Primer wurden auf einem DNA Synthesizer der Firma Applied Biosystems hergestellt, gelelektrophoretisch auf einem 20% Polyacrylamid/8M Harnstoff-Gel gereinigt und eine 5'Phosphatgruppe angeheftet. Dazu wurde 1nmol gereinigtes Oligonukleotid in 20μl Volumen mit 10 Einheiten T4 Polynukleotidkinase nach Vorschrift des Herstellers (Boehringer Mannheim) inkubiert. Anschließend wurde das Oligonukleotid mit Phenol ausgeschüttelt und Ethanol gefällt.

In einem Volumen von 10μl wurden 4pmol Oligonukleotid mit 0.1pmol partiell einzelsträngiger DNA gemischt und 5 min auf 65 °C erhitzt. Dannach werden 23μl H20 und 4μl Puffer (625mM KCl/275mM Tris(r) HCl pH7.5/150mM MgCl2/20mM DTT/0.5mM ATP/je 0.25mM dATP, dCTP, dGTP und dTTP), 4 Einheiten T4 DNA Ligase und 1 Einheit Klenow DAN-Polymerase (Boehringer Mannheim) dazugegeben. Diese Mischung wird für 45 min bei Raumtemperatur inkubiert. Bis zur Transformation wird die Probe auf Eis gelagert.

Zur Transformation wurden 10μl der zu transformierenden Proben mit 150 μl kompetenten E.coli Zellen (Cohen, S.N.; Chang A.C.Y.; Hsü, L. PNAS 69, 2110 (1972)) BMH 7118 mutS gemischt, für 30 min auf Eis gehalten und 3 min bei 42 °C inkubiert. Dannach wurde 1ml LB Medium dazugeben, für 1h bei 37 °C und 150 upm geschüttelt. 10ml LB + 100ug/ml Ampicillin Medium wurden mit diesem Transformationsgemisch angeimpft und über Nacht bei 37 °C geschüttelt.

Aus dieser Kultur wurde Plasmid DNA isoliert und in kompetente E. coli MK30-3 Zellen transformiert. Je 200μl des Transformationsgemisches wurden auf LB + 100ug/ml Ampicillin Platten ausgestrichen und über Nacht bei 37 °C inkubiert.

Zur Überprüfung der Konstrukte wird aus den erhaltenen Kolonien Plasmid DNA isoliert und mit entsprechenden Restriktionsenzymen gespalten. Die Ausbeute an gewünschten Mutanten lag zwischen 50% und 80%.

Für die Ligasereaktionen zur Herstellung aller benutzten Plasmide wurden jeweils 3 Teile der mit den entsprechenden Enzymen geschnittenen Insert DNA mit einem Teil gespalener Vektor DNA in 20μl Ligasepuffer (Endkonzentration: 10mM MgCl2/1mM DTT/ 50mM Tris(r)-HCl pH 7.5 1mM ATP) gemischt und nach Zugabe von 7.5 Einheiten T4 DNA Ligase über Nacht bei 8 °C inkubiert. Diese Ligationsgemische wurden wie beschrieben in kompetente E. coli Zellen oder entsprechend der in der deutschen Offenlegungsschrift DE 35 27 913 beschriebenen Vorgehensweise in Stämme der Gattung Bacillus transformiert. Zur Überprüfung der Konstrukte wurde aus den erhaltenen Kolonien DNA isoliert und mit geeigneten Restriktionsenzymen gespalten.

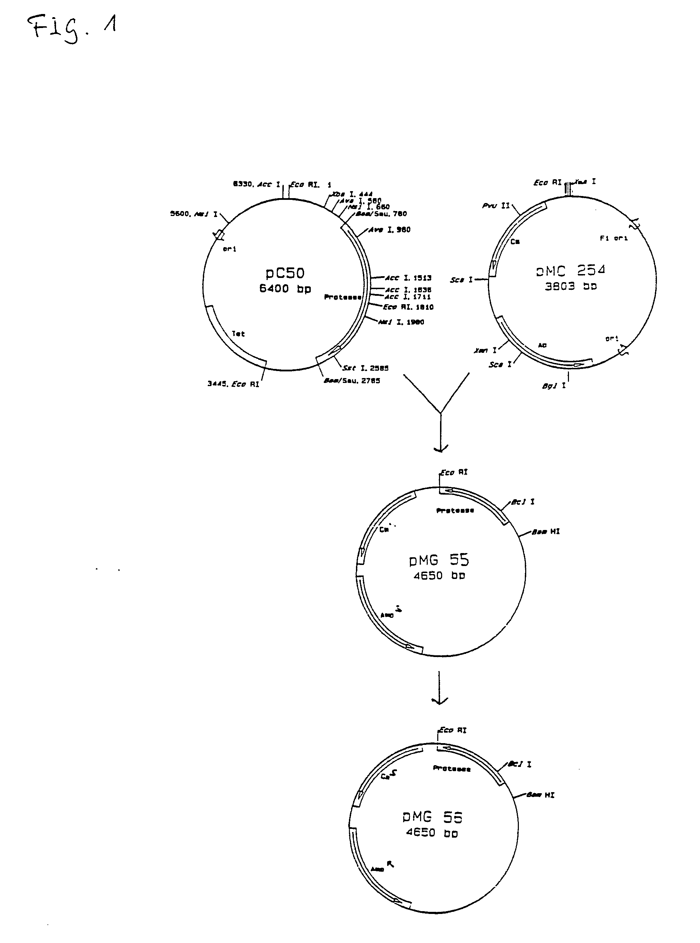

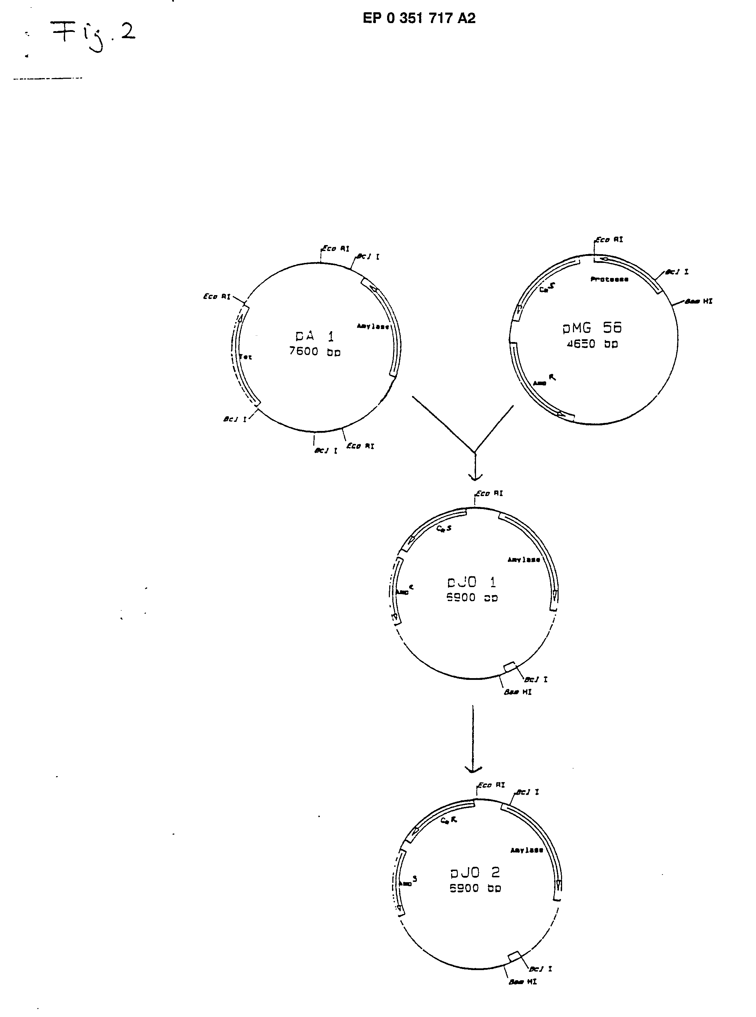

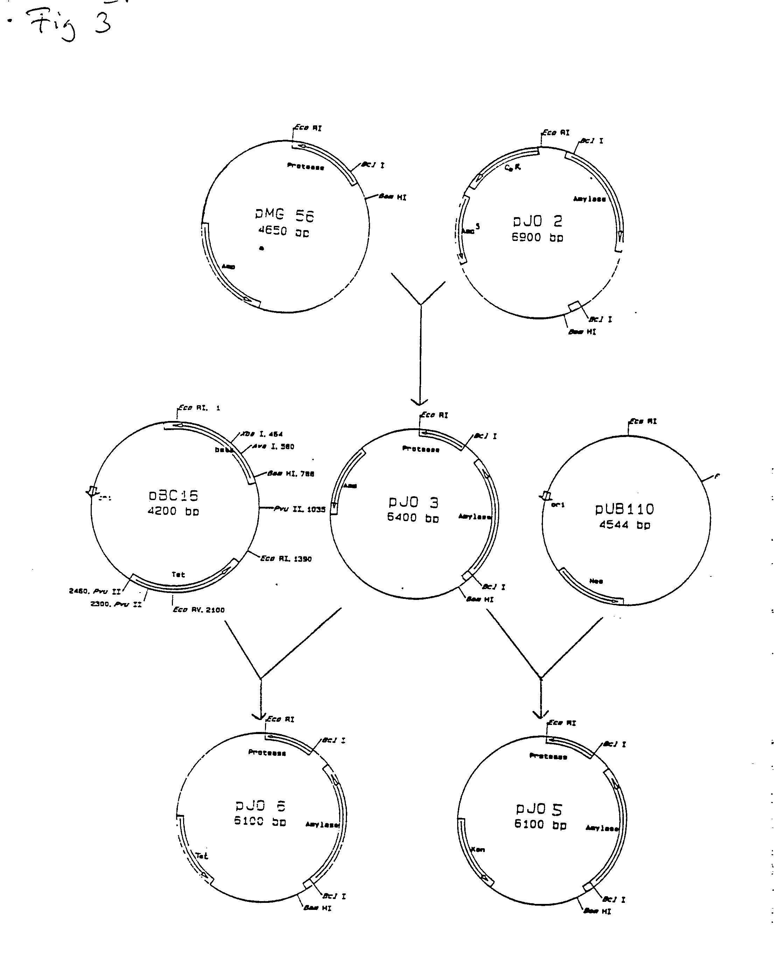

**Ansprüche**

1. In Bacillus exprimierbares Bacillus Plasmid, enthaltend einen Promotor für die Expression von Proteinen und in funktionsfähiger Weise damit verknüpft das Strukturgen eines Enzyms, dadurch gekennzeichnet, daß es einen Protease-Promotor verknüpft mit einem Amylase-Strukturgen enthält.

2. Plasmid nach Anspruch 1, dadurch gekennzeichnet, daß es als Protease-Promotor den Promotor von Subtilisin Carlsberg enthält.

3. Plasmid aus Bacillus licheniformis nach den Ansprüchen 1 oder 2, dadurch gekennzeichnet, daß es ein Strukturgen enthält, das nur für die thermostabile alpha-Amylase von Bacillus licheniformis, insbesondere Bacillus licheniformis DSM641 oder deren stärkespaltende Varianten codiert.

4. Plasmid nach einem der Ansprüche 1 bis 3, dadurch gekennzeichnet, daß es sowohl über eine EcoRI- als auch über eine BclI-Schnittstelle verfügt.

5. Plasmid nach einem der Ansprüche 1 bis 4, dadurch gekennzeichnet, daß es über einen oder mehrere Selektionsmarker, insbesondere Resistenzmarker für ein oder mehrer Antibiotica verfügt.

6. Plasmid nach einem der Ansprüche 1 bis 5, dadurch gekennzeichnet, daß es von dem Bacillus Plasmid pUB 110 abgeleitet ist.

7. Plasmid nach einem der Ansprüche 1 bis 6, dadurch gekennzeichnet, daß es von dem Bacillus Plasmid pBCE16 abgeleitet ist.

8. Neue Mikroorganismen der Gattung Bacillus licheniformis oder Bacillus subtilis, enthaltend Plasmide nach einem der Ansprüche 1 bis 7, mit der Fähigkeit, alpha-Amylase in der stationären Wachstumsphase zu erzeugen.

9. Verfahren zur Erzeugung einer alpha-Amylase, gewünschtenfalls in Abmischung mit alkalischer Protease, insbesondere mit Subtilisin Carlsberg, dadurch gekennzeichnet, daß Mikroorganismen nach Anspruch 8 unter den für die Erzeugung von Subtilisin Carlsberg üblichen Bedingungen kultiviert und das exocellulär erzeugte Enzym abgetrennt und aufgearbeitet wird.

Patentansprüche für folgende Vertragsstaaten: ES, GR

1. Verfahren zur Erzeugung einer alpha-Amylase, gewünschtenfalls in Abmischung mit alkalischer Protease, bei dem ein Mikroorganismus der Gattung Bacillus licheniformis oder Bacillis subtilis, welcher Plasmide mit der Fähigkeit, alpha-Amylase in der stationären Wachstumsphase zu erzeugen enthält, unter den für die Erzeugung von Subtilisin Carlsberg üblichen Bedingungen kultiviert und das exocellulär erzeugte Enzym abgetrennt und aufgearbeitet wird, wobei das in Bacillus exprimierbare Plasmid einen Promotor für die Expression von Proteinen und in funktionsfähiger Weise damit verknüpft das Strukturgen eines Enzyms enthält, dadurch gekennzeichnet, daß in dem im Mikroorganismus vorhandenen Plasmid ein Protease-Promotor verknüpft mit einem Amylase-Strukturgen vorhanden ist.

2. Verfahren nach Anspruch 1, dadurch gekennzeichnet, daß es als Protease-Promotor den Promotor von Subtilisin Carlsberg enthält.

3. Verfahren nach den Ansprüchen 1 oder 2, dadurch gekennzeichnet, daß es ein Strukturgen enthält, das nur für die thermostabile alpha-Amylase von Bacillus licheniformis, insbesondere Bacillus licheniformis DSM641 oder deren stärkespaltende Varianten codiert.

4. Verfahren nach einem der Ansprüche 1 bis 3, dadurch gekennzeichnet, daß es sowohl über eine EcoRI- als auch über eine BclI-Schnittstelle verfügt.

5. Verfahren nach einem der Ansprüche 1 bis 4, dadurch gekennzeichnet, daß es über einen oder mehrere Selektionsmarker, insbesondere Resistenzmarker für ein oder mehrer Antibiotica verfügt.

6. Verfahren nach einem der Ansprüche 1 bis 5, dadurch gekennzeichnet, daß es von dem Bacillus Plasmid pUB 110 abgeleitet ist.

7. Verfahren nach einem der Ansprüche 1 bis 6, dadurch gekennzeichnet, daß es von dem Bacillus Plasmid pBCE16 abgeleitet ist.

Fig. 1

Fig. 2

D 8002 EP

Fig 3